# EUROPEAN PATENT APPLICATION

(11) **EP 4 684 872 A1**
(43) Date of publication of application: **28.01.2026**
(21) Application number: 24191301.1
(22) Date of filing: 26.07.2024
(51) Int. Cl.: B01J 13/04

(54) **DEVICE AND METHOD FOR WATER-IN-AIR GENERATION OF DROPLETS**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to a device for water-in-air generation of droplets comprising a collection vessel, a droplet generation device positioned above the collection vessel comprising a piezoelectric transducer (21) and at least one capillary (22) with a nozzle (23) pointing downwards to the collection vessel; a laser source (30) generating a visible light laser beam (31) with tophat profile, and an imaging setup for continuously monitoring the droplet generation (40). Said device is particularly useful for preparing monodisperse giant unilamellar vesicles of any composition and three-dimensional microenvironment niches for cells or synthetic cells in high throughput.

## Description

The present invention relates to a device for water-in-air generation of droplets comprising a collection vessel, a droplet generation device positioned above the collection vessel comprising a piezoelectric transducer (21) and at least one capillary (22) with a nozzle (23) pointing downwards to the collection vessel; a laser source (30) generating a visible light laser beam (31) with tophat profile, and an imaging setup for continuously monitoring the droplet generation (40). Said device is particularly useful for preparing monodisperse giant unilamellar vesicles of any composition and three-dimensional microenvironment niches for cells or synthetic cells in high throughput.

### Background of the invention

Giant unilamellar vesicles (GUVs) with diameters comprised between 1 and 10 µm might offer great potential particularly for drug delivery, as they can carry massive quantities of active compounds as well as particles up to several micrometres in size. In contrast, lipid-based small unilamellar vesicles (SUVs), with diameters smaller than 100 nm, have only limited capacities for entrapping pharmaceutically active compounds, and cannot be charged efficiently with the pharmaceutically active compound.

Despite the growing demand for biomedical and synthetic biology applications based on giant unilamellar vesicles, the potential of giant unilamellar vesicles to deliver large and complex cargos into the intracellular space or, in general, their ability to interact with living cells have been only superficially explored.

High-throughput droplet generation technologies have enabled droplet-based microfluidics to flourish into an intersectional field where physics, chemistry, and biology interface in rapidly expanding application spaces. While the foundational use of PDMS-based microfluidic channel orientations produces a variety of water-in-oil droplet emulsions, there are recognized drawbacks to templating biology-focused water droplets within oils, especially when the biological cargo should be recovered as a functional aspect of an assay and towards the reduction of oils in the environment.

While some work has demonstrated biopolymer-based fluids that may be gelled using different methods when the desired output is a hydrogel particle thereby avoiding emulsification, these methods are not compatible with cell culture applications as they use harmful UV light to induce photopolymerization that causes DNA-damage to cells if incorporated as a natural cargo. Especially in the application of encapsulating stem cells as biological cargo, the synthetic environment enabled by photopolymerization of polymers to provide a blank-slate extracellular matrix scaffolding is desirable however, stem cells are extremely sensitive to their environment and exposure to harmful irradiation and chemical environments would have a negative impact on their viability, propensity to remain undifferentiated, and further ability to differentiate upon induction by controlled cues.

In a parallel field for the application of droplet emulsions, giant unilamellar vesicle (GUV) formation has characteristically been accomplished using water-in-oil emulsions produced in microfluidics devices or using continuous droplet interface crossing encapsulation (cDICE) apparatuses. However, the production of GUVs is notoriously cumbersome, inefficient, and highly dependent on uncontrollable variables that heavily decrease protocol reproducibility.

Although these drawbacks to the use of GUVs in synthetic biology and biotechnology processes have been dramatically improved and optimized, there still remains some challenges in the particular implementation of the chemistry and technology. The chemistry is derived using fluorinated-compounds that comprise both fluorinated oils used to create water-in-oil emulsions in microfluidics devices and that are stabilized and functionalized using fluorosurfactants. The use of these fluorinated oils has enabled high-throughput generation of GUVs as the chassis for the formation of synthetic cells in truly ground breaking concentrations with unique functionalities.

However, fluorinated compounds are considered to be non-biodegradable forever chemicals as there are currently no methods to breakdown or recycle these materials once generated.

Therefore, there is a demand for droplet generation devices that exploit the technical features of fluorosurfactants without the need to additionally use fluorinated oils.

Exisiting droplet generation devices of the prior art typically are restricted to a single size-range, specific application space, and are limited in phase chemistry orientations (i.e., water-in-oil, oil-in-water, and water-in-water droplets) due to constrained fundamental parameter spaces. These parameters include values associated with fluid dynamics, environmental factors for cargo loads like metabolic demands for maintaining cell viability or pH for chemical reactions etc., and limitations in the technical development for commercially available instrumentation. For example, hydrogel particles, lipid-derived aerosols, and water-in-water system with lipid compartments have been realizied (see Coyne, C. W.e tal. Jove-J Vis Exp 2014, (84); Stetten, A. Z. et al. J Colloid Interf Sci 2016, 484, 270-278.; Mitsche, M. A. et al. J Phys Chem 8 2010, 114 (9), 3276-3284; Lipowsky, R. et al. J Phys Chem B 2018, 122 (13), 3572-3586; Van de Cauter, L. et al Acs Synth Biol 2021, 10 (7), 1690-1702.) Principles commonly used in fluid dynamics printing technologies, design of cell sorting instrumentation used in the field of flow cytometry, and ultrasonic droplet generators including in-air microfluidics devices (Baumgartner, D. et al. Phys Rev Fluids 2020, 5 (10)) have been previously described discretely. However, examples are limited to one-off implementations with only few advanced instances to include software interfaces exploiting automation and machine learning.

Further, to operate with the flow rates, frequencies, and power requirements to achieve characterization of the complete parameter spaced needed for instrument automation, additional design considerations and iterations must be taken into account. Taken together, improved droplet generation devices suitable for diverse applications in synthetic biology and biotechnology are required.

Thus, it is the objective of the present invention to provide an improved device for droplet generation without using fluorinated oils, wherein said device is compatible with various droplet compositions needed for applications in synthetic biology and biotechnology.

The objective of the present invention is solved by the teachings of the independent claims. Further advantageous features, aspects and details of the invention are evident from the dependent claims, the description, the figures, and the examples of the present application.

### Brief description of the invention

In order to overcome the limitations of the prior art described above, the present invention provides methods and a modular device (1) for generating stable water droplets in-air. The inventive droplet generation device (1) can be used with a variety of biomolecule-containing solutions having a broad range of fluid dynamics properties (e.g. viscosity). The versatile use of the inventive device (1) is enabled by the ability to modulate the amplitude of the acoustic sine wave controlling droplet formation by the piezoelectric transducer (21) thereby maintaining optimized control over flow-rate and frequency combinations by high-tolerance fluidic channel designs. The device further allows the production of droplets in preferred size ranges (i.e., 5-100 µm diameters) by using nozzle diameters that enable hydrodynamic focusing, and droplet time-of-flight through a tophat laser line that uses a wavelength of light compatible with biology.

The present invention also provides methods for forming GUVs by using phase transitions from air-water to water-water interfaces, thereby removing the need for oils in the formation method. The present invention also provides methods for forming three-dimensional microenvironment niches using photosensitive chemistry, wherein water-in-water droplet phase orientations are implemented that are stabilized using fluorosurfactants. Applications of the synthetic three-dimensional microenvironment niches may include demonstration of maintenance of stem cell pluripotency, controlled activation of stem cell differentiation signaling pathways, investigations of cell-synthetic cell interactions in 3D towards templating synthetic tissues for biomaterials applications, and overall optimization of synthetic three-dimensional microenvironment niches for cell biology applications.

Thus, the present invention is directed to a device (1) for water-in-air generation of droplets comprising:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10), and
wherein the laser beam (31) is aligned and focused on said flow of droplets passing from the nozzle (23) to the collection vessel (10) to induce a photochemical reaction in the droplets.

The device (1) according to the invention is constructed such that alignment of the droplet generation device (20), imaging setup (40), laser line (31), and collection vessel (10) can be controlled by manual micromanipulation. Differential flow rates are set and characterized with the application of an acoustic field for frequency and amplitude by monitoring droplet generation using stroboscopic imaging. The acoustic field created by the piezoelectric transducer (21) is adjusted according to the droplet generation monitored by the imaging setup (40). Thus, when droplet formation is disturbed or satellite drops are formed due to a change in viscosity the amplitude of the acoustic field created by the piezoelectric transducer (21) is adjusted to restore / maintain / improve droplet generation at a high frequency.

In a preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, and wherein the droplet generation device (20) is mounted on a self-centering mount (50).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, and wherein the device (1) further comprises a liquid delivery system (60) comprising a pump system (61) and at least one liquid reservoir (62), wherein the liquid delivery system (60) delivers liquid(s) from the at least one liquid reservoir (62) to the at least one capillary (22).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, and wherein the piezoelectric transducer (21) is designed as a vertically arranged tube surrounding the at least one capillary (22) with the nozzle (23), thereby the nozzle (23) is located in an acoustic field generated by the piezoelectric transducer (21).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, and wherein the device (1) further comprises a feedback controller (70) operatively coupled to the imaging setup for continuous monitoring the droplet generation (40) and the piezoelectric transducer (21), wherein the feedback controller (70) is configured to control the piezoelectric transducer (21).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, and wherein the vertical distance d between the nozzle (23) and the collection vessel (10) is large enough to allow volatile components to evaporate from the flow of droplets, preferably at least 40 mm.

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, and wherein the at least one capillary (22) comprises a first capillary (22a) with a first cavity (24a) and a second capillary (22b) with a second cavity (24b), wherein the second capillary (22b) has a smaller diameter than the the first capillary (22a) and the second capillary (22b) is nested in the first capillary (22a).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, and wherein the collection vessel (10) is mounted on a rotation mount (80), wherein the rotational speed of the rotation mount (80) is adjustable to generate a rotational laminar flow profile of a collection liquid in the collection vessel (10) towards the edge of the collection vessel which is synchronized with the droplet flow rate.

A further aspect is directed to a method for preparing monodisperse giant unilamellar vesicles using the device (1) for water-in-air generation of droplets according to the invention. Thus, the present invention is also directed to a method for preparing monodisperse giant unilamellar vesicles using the device (1) for water-in-air generation of droplets comprising the following steps:
a) providing a core-annular liquid-liquid flow comprising an aqueous core solution and a water-immiscible organic shell solution, wherein the water-immiscible organic shell solution comprises a volatile organic solvent and a first lipid,
b) acoustically modulating the core-annular liquid-liquid flow, thereby forming a flow of core-shell droplets comprising a core consisting of the aqueous core solution and a shell consisting of the water-immiscible organic shell solution, wherein the first lipid forms a monolayer around the droplet core,
c) at least partially evaporating the volatile organic solvent from the flow of core-shell droplets,
d) submerging the flow of core-shell droplets in a rotating dispersion to produce monodisperse giant unilamellar vesicles,

wherein in step d) the rotating dispersion comprises an aqueous phase and a second lipid, wherein the second lipid is arranged as a monolayer on the surface of the aqueous phase so that the monolayer of the second lipid encloses the core-shell droplets thereby forming a double layer with the monolayer of the first lipid, and
wherein in step d) the rotating dispersion exhibits a rotational laminar flow profile which is synchronized with the flow rate of the core-shell droplets.

In a preferred embodiment, the method for preparing monodisperse giant unilamellar vesicles using the device (1) for water-in-air generation of droplets comprises the following steps:
a) providing a core-annular liquid-liquid flow comprising an aqueous core solution and a water-immiscible organic shell solution, wherein the water-immiscible organic shell solution comprises a volatile organic solvent and a first lipid,
b) acoustically modulating the core-annular liquid-liquid flow, thereby forming a flow of core-shell droplets comprising a core consisting of the aqueous core solution and a shell consisting of the water-immiscible organic shell solution, wherein the first lipid forms a monolayer around the droplet core,
c) at least partially evaporating the volatile organic solvent from the flow of core-shell droplets,
d) submerging the flow of core-shell droplets in a rotating dispersion to produce monodisperse giant unilamellar vesicles,

wherein in step d) the rotating dispersion comprises an aqueous phase and a second lipid, wherein the second lipid is arranged as a monolayer on the surface of the aqueous phase so that the monolayer of the second lipid encloses the core-shell droplets thereby forming a double layer with the monolayer of the first lipid, and
wherein in step d) the rotating dispersion exhibits a rotational laminar flow profile which is synchronized with the flow rate of the core-shell droplets, and wherein the monodisperse giant unilamellar vesicles have a diameter measured by confocal microscopy between 5 µm and 30 µm.

In a preferred embodiment, the method for preparing monodisperse giant unilamellar vesicles using the device (1) for water-in-air generation of droplets comprises the following steps:
a) providing a core-annular liquid-liquid flow comprising an aqueous core solution and a water-immiscible organic shell solution, wherein the water-immiscible organic shell solution comprises a volatile organic solvent and a first lipid,
b) acoustically modulating the core-annular liquid-liquid flow, thereby forming a flow of core-shell droplets comprising a core consisting of the aqueous core solution and a shell consisting of the water-immiscible organic shell solution, wherein the first lipid forms a monolayer around the droplet core,
c) at least partially evaporating the volatile organic solvent from the flow of core-shell droplets,
d) submerging the flow of core-shell droplets in a rotating dispersion to produce monodisperse giant unilamellar vesicles,

wherein in step d) the rotating dispersion comprises an aqueous phase and a second lipid, wherein the second lipid is arranged as a monolayer on the surface of the aqueous phase so that the monolayer of the second lipid encloses the core-shell droplets thereby forming a double layer with the monolayer of the first lipid, and
wherein in step d) the rotating dispersion exhibits a rotational laminar flow profile which is synchronized with the flow rate of the core-shell droplets, and wherein the monodisperse giant unilamellar vesicles are prepared in a frequency between 5,000 vesicles per second and 30,000 vesicles per second.

In a preferred embodiment, the method for preparing monodisperse giant unilamellar vesicles using the device (1) for water-in-air generation of droplets comprises the following steps:
a) providing a core-annular liquid-liquid flow comprising an aqueous core solution and a water-immiscible organic shell solution, wherein the water-immiscible organic shell solution comprises a volatile organic solvent and a first lipid,
b) acoustically modulating the core-annular liquid-liquid flow, thereby forming a flow of core-shell droplets comprising a core consisting of the aqueous core solution and a shell consisting of the water-immiscible organic shell solution, wherein the first lipid forms a monolayer around the droplet core,
c) at least partially evaporating the volatile organic solvent from the flow of core-shell droplets,
d) submerging the flow of core-shell droplets in a rotating dispersion to produce monodisperse giant unilamellar vesicles,

wherein in step d) the rotating dispersion comprises an aqueous phase and a second lipid, wherein the second lipid is arranged as a monolayer on the surface of the aqueous phase so that the monolayer of the second lipid encloses the core-shell droplets thereby forming a double layer with the monolayer of the first lipid, and wherein in step d) the rotating dispersion exhibits a rotational laminar flow profile which is synchronized with the flow rate of the core-shell droplets, and
wherein the first lipid and the second lipid are independently of each other selected from the group consisting of:
   a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
   an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidyl inositol phosphates, cardiolipins, bis(monoacylglycero)phosphate derivatives;
   a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-dioleoyl-3-dimethylammonium-propane;
   a pH-sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-snglycero-3-succinate, N-palmitoyl homocysteine;
   a photoswitchable lipid;
   acylglycine derivatives, prenol derivatives, prostaglandine derivatives, glyco-sylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
   one of the aforementioned lipids coupled to a functional ligand selected from biotin, N-hydroxysuccinimide (NHS) ester, sulfo- NHS ester, nitrilotriacetic acid -nickel, amine, carboxylic acid, maleimides, dithiopyridinyl, pyridyl disulfide, pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator; and
   one of the aforementioned lipids coupled to polyethyleneglycol with a molecular weight comprised between 350 and 50,000 g/mol.

Another aspect is directed to a method for preparing a three-dimensional microenvironment niche for a cell or a synthetic cell using the device (1) for water-in-air generation of droplets according to the invention. Thus, the present invention is also directed to a method for preparing a three-dimensional microenvironment niche for a cell or a synthetic cell using the device (1) for water-in-air generation of droplets comprising the following steps:
a) providing a liquid flow comprising a cell and/or a synthetic cell, and a first cell culture medium comprising a photocrosslinkable polymer hydrogel, and a photocatalyst,
b) acoustically modulating the liquid flow, thereby forming a flow of droplets comprising the cell and/or the synthetic cell encapsulated in the first cell culture medium,
c) irradiating the droplets to photochemically induce a crosslinking of the photocrosslinkable polymer hydrogel thereby gelling the droplet
d) collecting the flow of gelled droplets in a second cell culture medium to produce a three-dimensional microenvironment niche.

In a preferred embodiment, the method for preparing a three-dimensional microenvironment niche for a cell or a synthetic cell using the device (1) for water-in-air generation of droplets comprises the following steps:
a) providing a liquid flow comprising a cell and/or a synthetic cell, and a first cell culture medium comprising a photocrosslinkable polymer hydrogel, and a photocatalyst,
b) acoustically modulating the liquid flow, thereby forming a flow of droplets comprising the cell and/or the synthetic cell encapsulated in the first cell culture medium,
c) irradiating the droplets to photochemically induce a crosslinking of the photocrosslinkable polymer hydrogel thereby gelling the droplet
d) collecting the flow of gelled droplets in a second cell culture medium to produce a three-dimensional microenvironment niche,
wherein in step d) the second cell culture medium is agitated.

In a preferred embodiment, the method for preparing a three-dimensional microenvironment niche for a cell or a synthetic cell using the device (1) for water-in-air generation of droplets comprises the following steps:
a) providing a liquid flow comprising a cell and/or a synthetic cell, and a first cell culture medium comprising a photocrosslinkable polymer hydrogel, and a photocatalyst,
b) acoustically modulating the liquid flow, thereby forming a flow of droplets comprising the cell and/or the synthetic cell encapsulated in the first cell culture medium,
c) irradiating the droplets to photochemically induce a crosslinking of the photocrosslinkable polymer hydrogel thereby gelling the droplet
d) collecting the flow of gelled droplets in a second cell culture medium to produce a three-dimensional microenvironment niche,
wherein in wherein the photocrosslinkable polymer hydrogel is a polymer chemically functionalized with one or more methacrylate groups selected from the group consisting of: hyaluronic acid, chitosan, dextran, heparin, alginate, gelatin, fibrin, collagen type I, collagen type II, collagen type III, collagen type IV, fibronectin, vitronectin, tropoelastin, laminin, silk, sericin, polyvinyl alcohol, polyethylene glycol, sodium polyacrylate, acrylate polymers and copolymers thereof.

In a preferred embodiment, the method for preparing a three-dimensional microenvironment niche for a cell or a synthetic cell using the device (1) for water-in-air generation of droplets comprises the following steps:
a) providing a liquid flow comprising a cell and/or a synthetic cell, and a first cell culture medium comprising a photocrosslinkable polymer hydrogel, and a photocatalyst,
b) acoustically modulating the liquid flow, thereby forming a flow of droplets comprising the cell and/or the synthetic cell encapsulated in the first cell culture medium,
c) irradiating the droplets to photochemically induce a crosslinking of the photocrosslinkable polymer hydrogel thereby gelling the droplet
d) collecting the flow of gelled droplets in a second cell culture medium to produce a three-dimensional microenvironment niche,
wherein the photocatalyst is a Ru(II) complex.

### Definitions

Liposomes are composed of a lipid bilayer separating an aqueous internal compartment from the bulk aqueous phase. A liposome is a spherical vesicle having at least one lipid bilayer. The major types of liposomes are the multilamellar vesicles (MLV, with several lamellar phase lipid bilayers), the small unilamellar liposome vesicles (SUV, with one lipid bilayer), the large unilamellar vesicles (LUV), and the giant unilamellar vesicles (GUV).

Small unilamellar vesicles, large unilamellar vesicles and giant unilamellar vesicles are usually spherical with a diameter of typically 25 to 50 nm for small unilamellar vesicles, with a diameter of typically more than 50 to 1,000 nm for large unilamellar vesicles, and with a diameter of typically more than 1 to 1,000 µm for giant unilamellar vesicles. However, the chemical and mechanical instabilities of unsaturated fatty acids under high ionic strength conditions, especially multivalent cations, and their sensitivity to pH changes are considered to be the main challenges in utilizing these vesicles. In addition, inserting molecules into these vesicles represents a particular challenge given their impermeability and mechanical instability.

The term **"vesicle"** refers to a non-natural or synthetic membranous and usually fluid-filled pouch resulting from the supramolecular assembly of lipids including, but not limited to, phospholipids. The interior contents of a phospholipid vesicle are separated from the exterior surroundings by at least one phospholipid bilayer. A phospholipid bilayer is a sheet of lipids two molecules thick, arranged so that the hydrophilic phosphate heads point "out" to the solution on either side of the bilayer and the hydrophobic tails point "in" to the core of the bilayer. This results in two "leaflets" which are each a single molecular layer of phospholipids. A "unilamellar vesicle" refers to a vesicle comprising only one phospholipid bilayer; "multilamellar vesicle" refers to a vesicle comprising more than one phospholipid bilayer. A "symmetric bilayer" is defined as a bilayer possessing two leaflets of the same composition, whereas an "asymmetric bilayer" is defined as a bilayer possessing two leaflets which differ in composition.

The vesicle of the present invention can have a unilamellar bilayer including a lipid and a component integrated by the bilayer. The integrated component can be a protein or a fragment thereof, a peptide, a polypeptide, an enzyme or a fragment thereof. Exemplary proteins are ATPase, E-cadherins, integrins, alpha-haemolisin. An exemplary polypeptide is the antibiotic gramicidin.

A **"water-based droplet"** is a droplet, which contains water or a dispersion of any substance in water. Also, a water-based droplet is a droplet, which consists of water or of a dispersion of any substance in water. More specifically, a water-based droplet in the sense of the present invention is a droplet which is composed of water including salts and the at least one lipid or at least one polymer such as those described for the synthetic niche formation.

**"Giant unilamellar vesicles"** are unilamellar vesicles, preferably spherical, and with a diameter comprised between 1 µm and 100 µm. The diameter of giant unilamellar vesicles is preferably measured by confocal microscopy using the imgaging setup (40).

The term **"core-annular"** droplet as used herein refers to a droplet which is generated as a consequence of the two-channel acoustofluidic device design. In this design, the core fluid injected into the central channel comprises a water-based solution. This core channel is nested within a second channel and injects the sheath solution comprising a volatile solvent with at least one lipid in suspension. When the two fluids exit the nesting channels and the fluid flow is acoustically actuated, a so-called core-annular droplet profile is initially generated, whereby the at least one lipid type within the sheath solution may start to assemble at the interface as the volatile solvent begins to evaporate.

The term "giant" as used herein refers to unilamellar vesicles which form a lipid compartment on the mesoscale.

The terms **"cell delivery", "cell-targeting", "delivery", "cell type specific delivery"** or **"targeted delivery"** are used interchangeably herein to refer to the delivery of particular agents or compounds or macromolecules by the giant unilamellar vesicles to regions, tissues or cells, intracellularly or extracellularly, in *vivo* or *in vitro.*

When the delivery occurs in the extracellular space of the cells, it means that the agent or compound or macromolecule is brought at a sufficient small distance to the target cell to allow interaction of the agent or compound or macromolecule with said cell, and thus the desired effect on said cell.

The agent or compound or macromolecule can be present on the surface of the giant unilamellar vesicles or in the lumen of giant unilamellar vesicles.

The term "a first lipid" as used herein refers to one lipid or the lipid per discrete fluid in the mechanism, or one lipid or the lipid in the droplet phase and one other lipid or the other lipid in the collection phase. In case a lipid combination is used which could contain up to 6 different lipids where some lipids are fluorescent and others contain bio-functionalizable chemical head groups, a first lipid refers to one lipid of this combination.

The term **"photoswitchable lipid"** as used herein refers to lipids which have a light-sensitive moiety such as an azobenzene that changes molecular configuration when irradiated by specific wavelengths of light. The conformational change in the molecule is used to modulate membrane biophysics.

The term **"laser source"** refers preferably to a 450 nm laser source as this is the wavelength of light needed for the photochemical activation of Ru(II) compounds. The term laser source can also apply to any laser with wavelengths in the visible spectrum, between 400 nm and 900 nm that.

The term **"tophat profile"** as used herein defines an intensity profile of the laser which is flat and uniform with sharp edges and where the energy drops to zero rapidly within a defined width vs length profile. Consequently, a "tophat laser line" is a laser beam with a tophat profile.

The term **"synchronized"** as used herein refers to the type of electronic connection used to control the piezeoelectric element and the back-light LED strip.

The piezeoelectric element and the back-light LED strip are each connected to a dual-channel waveform generator that is programmed to modulate the output of each channel in parallel with matching waveforms up to MHz freqencies (with a range of function shapes and phases possible) but with discrete amplitudes up to 1000 Vpp when the signal is directed through an electronic high voltage amplifier. The signal through each channel connected to either the piezeoelectric element or the back-light LED strip is then synchronized, in phase, with discrete amplitudes up to 1000 Vpp with an amplifier with a range of frequencies between 1 and 100 kHz depending on the desired specifications for the produced droplets. Synchronization is not applied to the tophat laser line profile. Synchronization is used to create a stroboscopic phenomenon that enables the flow stream of droplets to be captured by a high-speed camera in the image relay path. This is required as the droplet generation frequency is faster than resolvable by the human eye and standard imaging setups.

The terms **"protein"** and **"peptide"** are used interchangeably herein to refer to a polymer of amino acid residues. These terms apply to naturally occurring amino acid polymers and non-naturally occurring amino acid polymers, as well as to amino acid polymers in which one or more amino acid residues are an artificial chemical mimetic of a corresponding naturally occurring amino acid. As used herein, the terms encompass amino acid chains of any length, including full-length proteins, wherein the amino acid residues are linked by covalent peptide bonds, and fragments thereof.

The terms "nucleic acid", "oligonucleotide," and "polynucleotide" refer to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides.

The term **"volatile"** refers to organic solvents such as acetonitrile, nitromethane, piperidine, sulfolane, dimethylcarbonate, ethylencarbonate, dimethylsulfoxide, ether such as tetrahydrofurane (THF), diethylether, ketones such as acetone, butanone or pentanone, amides such as dimethylformamide (DMF) or dimethylacetamide, halogenated solvents such as chloroform, methylene chloride, tetrachloromethane, carboxylic acid ester such as acetic acid ethyl ester, alcohols such as methanol, ethanol, propanol, isopropanol, carboxylic acids such as formic acid, acetic acid, propionic acid, butyric acid hexyl ester, formamide, petrolether, ethers such as methyl-tert.-butylether (MTDC) or dioxane, hydrocarbons such as hexane, pentane or cyclohexane, aromatic solvents such as toluene, benzene, chlorobenzene, nitrobenzene, aniline, pyridine, anisol or xylene. Volatile organic solvents have preferably a boiling point below 215°C.

### "Fluid dynamics parameters"

The relevant fluid dynamics parameters that comprise the look up table for acoustic droplet generation includes the following parameters, which in the case of two-channel devices then takes a multiparametric input to use machine vision feedback control to predict the operating conditions for desired droplet generation:
1. Viscosity (µ) of the fluids, both dynamic and kinematic
2. Surface tension (σ) or interfacial tension (γ) of the fluids, influencing droplet formation and stability at the fluid surface
3. Density (ρ) of the fluids which impacts on the droplet dymanics with respect to interaction with acoustic waves and required amplitudes
4. Volumetric flow rate (mL/min) of the fluids through the capillary nozzle which is a determining parameter for the size and frequency of the produced droplets
5. Fluid velocity (v) which is the speed of the fluid in the capillary and directly impacts on the droplet formation dynamics including stability in air and during collection
6. Piezoelectric actuation frequency of the device (λ), that directly influences the acoustic waves generated and thereby the droplet formation dynamics
7. Piezoelectric amplitude which can be used to directly influence the size of the produced droplets as it depends on density and viscosity of the fluids
8. Reynold Number (Re) calculated based on the fluid velocity, characteristic length scale such as the diameter of the capillary nozzle, and kinematic viscosity
9. Capillary Number (Ca) calculated knowing the ratio of viscous forces to surface tension forces to favor the generation of more complex interfaces, defined as viscosity * fluid velocity divided by density
10. Weber Number (We) calculated knowing the ratio of inertial forces to surface tension forces and given by density * fluid velocity^2 * nozzle diameter divided by density

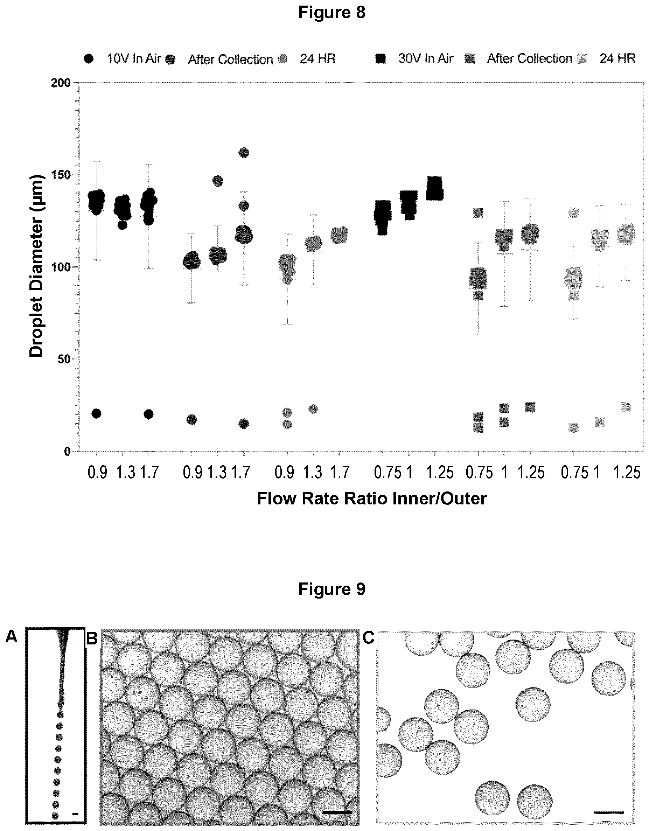

When parameters 8, 9, and 10 are within determined ranges for different fluid specifications of parameters 1, 2, and 3, then optimal operating parameters 4, 5, 6, and 7 can be predicted to produce droplets with desired size and frequency. Figure 8 gives the basis for how these parameters are practically applied.

**"Aqueous mixture"** or **"aqueous phase"** or **"water phase"** refers to a solution or suspension of lipids or other molecules substantially in water. The aqueous mixtures are immiscible with the oil-based mixtures of the present invention.

**"Oil mixture"** or **"oil phase"** refers to a solution or suspension of lipids or other molecules in perfluorated water-immiscible solvents. Exemplary water-immiscible solvents, referred to as "oils," suitable for the preparation of certain lipid membranes and vesicles in the present invention include, but are not limited to, HFE-7000 (1,1,1,2,2,3,3-heptafluoro-3-methoxy-propane), methylnonafluor-n-butylethers, HFE-7100 (mixture of the isomers [methy nonafluoroisobutyl ether and methyl non-afluorobutyl ether] 1,1,1,2,2,3,3,4,4-nonafluoro-4-methoxy-butane and 1,1,1,2,3,3-hexafluoro-3-methoxy-2-(trifluoromethyl)propane), HFE-7200 (mixture of the isomers 1-ethoxy-1,1,2,2,3,3,4,4,4-nonafluorobutane and 1-ethoxy-1,1,2,3,3,3-hexafluoro-2-(trifluoromethyl)propane), HFE-7300 (1,1,1,2,2,3,4,5,5,5-decafluoro-3-methoxy-4-(trifluoromethyl)pentane), and HFE-7500 (3-ethoxy-1,1,1,2,3,4,4,5,5,6,6,6-dodecafluoro-2-(trifluoromethyl)hexane), FC-40, One of skill in the art will appreciate that other solvents are useful as the oil mixture in the present invention.

The **"polymer shell"** of the giant unilamellar vesicles is made of an amphiphilic copolymer having a lipophilic end arranged at the outer side and a hydrophilic end arranged at the inner side of the polymer shell. The structure is described in details in the paragraphs above.

The term **"monodisperse"** or **"monodispersed"** refers to vesicles or droplets of uniform size in a dispersed phase, where the vesicles or droplets are present in an unaggregate and discrete state. The dispersed phase according to the invention is preferably a water phase for the polymer shell stabilized giant unilamellar vesicles. **"Dispersed phase"** is the droplet phase whereas continuous phase would be the surrounding oil, volatile solvent, or even air before droplets are collected into solution.

Vesicles of "uniform size" means that the polymer shell stabilized giant unilamellar vesicles obtained after splitting, as well as the giant unilamellar vesicles released from the polymer shell, show a coefficient of variation in size lower than 16 %, lower than 15 %, lower than 14 %, lower than 13 %, lower than 12 %, lower than 11 %, lower than 10 %.

### Description of the invention

The present invention is directed to a device (1) for water-in-air generation of droplets comprising:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);
wherein the piezoelectric transducer (21) is configured to acoustically generate a flow of droplets passing from the nozzle (23) to the collection vessel (10), and wherein the laser beam (31) is aligned and focused on said flow of droplets passing from the nozzle (23) to the collection vessel (10) to induce a photochemical reaction in the droplets.

In a preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, and wherein the droplet generation device (20) is mounted on a self-centering mount (50).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, and wherein the device (1) further comprises a liquid delivery system (60) comprising a pump system (61) and at least one liquid reservoir (62), wherein the liquid delivery system (60) delivers liquid(s) from the at least one liquid reservoir (62) to the at least one capillary (22).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, wherein the droplet generation device (20) is mounted on a self-centering mount (50), and wherein the device (1) further comprises a liquid delivery system (60) comprising a pump system (61) and at least one liquid reservoir (62), wherein the liquid delivery system (60) delivers liquid(s) from the at least one liquid reservoir (62) to the at least one capillary (22).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, and wherein the piezoelectric transducer (21) is designed as a vertically arranged tube surrounding the at least one capillary (22) with the nozzle (23), thereby the nozzle (23) is located in an acoustic field generated by the piezoelectric transducer (21).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, wherein the piezoelectric transducer (21) is designed as a vertically arranged tube surrounding the at least one capillary (22) with the nozzle (23), thereby the nozzle (23) is located in an acoustic field generated by the piezoelectric transducer (21), and wherein the device (1) further comprises a liquid delivery system (60) comprising a pump system (61) and at least one liquid reservoir (62), wherein the liquid delivery system (60) delivers liquid(s) from the at least one liquid reservoir (62) to the at least one capillary (22).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, wherein the piezoelectric transducer (21) is designed as a vertically arranged tube surrounding the at least one capillary (22) with the nozzle (23), thereby the nozzle (23) is located in an acoustic field generated by the piezoelectric transducer (21), wherein the droplet generation device (20) is mounted on a self-centering mount (50), and wherein the device (1) further comprises a liquid delivery system (60) comprising a pump system (61) and at least one liquid reservoir (62), wherein the liquid delivery system (60) delivers liquid(s) from the at least one liquid reservoir (62) to the at least one capillary (22).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10), wherein the laser beam (31) is aligned and focused on said flow of droplets passing from the nozzle (23) to the collection vessel (10) to induce a photochemical reaction in the droplets, and wherein the device (1) further comprises a feedback controller (70) operatively coupled to the imaging setup for continuous monitoring the droplet generation (40) and the piezoelectric transducer (21), wherein the feedback controller (70) is configured to control the piezoelectric transducer (21).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile,
   and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, wherein the piezoelectric transducer (21) is designed as a vertically arranged tube surrounding the at least one capillary (22) with the nozzle (23), thereby the nozzle (23) is located in an acoustic field generated by the piezoelectric transducer (21), wherein the device (1) further comprises a liquid delivery system (60) comprising a pump system (61) and at least one liquid reservoir (62), wherein the liquid delivery system (60) delivers liquid(s) from the at least one liquid reservoir (62) to the at least one capillary (22), and wherein the device (1) further comprises a feedback controller (70) operatively coupled to the imaging setup for continuous monitoring the droplet generation (40) and the piezoelectric transducer (21), wherein the feedback controller (70) is configured to control the piezoelectric transducer (21).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, wherein the piezoelectric transducer (21) is designed as a vertically arranged tube surrounding the at least one capillary (22) with the nozzle (23), thereby the nozzle (23) is located in an acoustic field generated by the piezoelectric transducer (21), wherein the droplet generation device (20) is mounted on a self-centering mount (50),and wherein the device (1) further comprises a feedback controller (70) operatively coupled to the imaging setup for continuous monitoring the droplet generation (40) and the piezoelectric transducer (21), wherein the feedback controller (70) is configured to control the piezoelectric transducer (21).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, wherein the piezoelectric transducer (21) is designed as a vertically arranged tube surrounding the at least one capillary (22) with the nozzle (23), thereby the nozzle (23) is located in an acoustic field generated by the piezoelectric transducer (21), wherein the droplet generation device (20) is mounted on a self-centering mount (50), wherein the device (1) further comprises a liquid delivery system (60) comprising a pump system (61) and at least one liquid reservoir (62), wherein the liquid delivery system (60) delivers liquid(s) from the at least one liquid reservoir (62) to the at least one capillary (22), and wherein the device (1) further comprises a feedback controller (70) operatively coupled to the imaging setup for continuous monitoring the droplet generation (40) and the piezoelectric transducer (21), wherein the feedback controller (70) is configured to control the piezoelectric transducer (21).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, and wherein the vertical distance d between the nozzle (23) and the collection vessel (10) is large enough, i.e. preferably at least 40 mm, to allow volatile components to evaporate from the flow of droplets, preferably at least 40 mm.

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, and
wherein the at least one capillary (22) comprises a first capillary (22a) with a first cavity (24a) and a second capillary (22b) with a second cavity (24b), wherein the second capillary (22b) has a smaller diameter than the the first capillary (22a) and the second capillary (22b) is nested in the first capillary (22a).

In a further preferred embodiment, the device (1) for water-in-air generation of droplets comprises:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel;
- a laser source (30) generating a visible light laser beam (31) with tophat profile, and
- an imaging setup for continuous monitoring the droplet generation (40) comprising a high-speed camera (41), a microscope lens (42) and a backlight (43);

wherein the piezoelectric transducer (21) is configured to acoustically generate a flow of droplets passing from the nozzle (23) to the collection vessel (10),
wherein the laser beam (31) is aligned and focused on said flow of droplets passing from the nozzle (23) to the collection vessel (10) to induce a photochemical reaction in the droplets, and wherein the collection vessel (10) is mounted on a rotation mount (80), wherein the rotational speed of the rotation mount (80) is adjustable to generate a rotational laminar flow profile of a collection liquid in the collection vessel (10) towards the edge of the collection vessel which is synchronized with the droplet flow rate.

A further aspect is directed to a method for preparing monodisperse giant unilamellar vesicles using the device (1) for water-in-air generation of droplets according to the invention. Thus, the present invention is also directed to a method for preparing monodisperse giant unilamellar vesicles using the device (1) for water-in-air generation of droplets comprising the following steps:
a) providing a core-annular liquid-liquid flow comprising an aqueous core solution and a water-immiscible organic shell solution, wherein the water-immiscible organic shell solution comprises a volatile organic solvent and a first lipid,
b) acoustically modulating the core-annular liquid-liquid flow, thereby forming a flow of core-shell droplets comprising a core consisting of the aqueous core solution and a shell consisting of the water-immiscible organic shell solution, wherein the first lipid forms a monolayer around the droplet core,
c) at least partially evaporating the volatile organic solvent from the flow of core-shell droplets,
d) submerging the flow of core-shell droplets in a rotating dispersion to produce monodisperse giant unilamellar vesicles,

wherein in step d) the rotating dispersion comprises an aqueous phase and a second lipid, wherein the second lipid is arranged as a monolayer on the surface of the aqueous phase so that the monolayer of the second lipid encloses the core-shell droplets thereby forming a double layer with the monolayer of the first lipid, and
wherein in step d) the rotating dispersion exhibits a rotational laminar flow profile which is synchronized with the flow rate of the core-shell droplets.

In a preferred embodiment, the method for preparing monodisperse giant unilamellar vesicles using the device (1) for water-in-air generation of droplets comprises the following steps:
a) providing a core-annular liquid-liquid flow comprising an aqueous core solution and a water-immiscible organic shell solution, wherein the water-immiscible organic shell solution comprises a volatile organic solvent and a first lipid,
b) acoustically modulating the core-annular liquid-liquid flow, thereby forming a flow of core-shell droplets comprising a core consisting of the aqueous core solution and a shell consisting of the water-immiscible organic shell solution, wherein the first lipid forms a monolayer around the droplet core,
c) at least partially evaporating the volatile organic solvent from the flow of core-shell droplets,
d) submerging the flow of core-shell droplets in a rotating dispersion to produce monodisperse giant unilamellar vesicles,

wherein in step d) the rotating dispersion comprises an aqueous phase and a second lipid, wherein the second lipid is arranged as a monolayer on the surface of the aqueous phase so that the monolayer of the second lipid encloses the core-shell droplets thereby forming a double layer with the monolayer of the first lipid, and
wherein in step d) the rotating dispersion exhibits a rotational laminar flow profile which is synchronized with the flow rate of the core-shell droplets, and
wherein the monodisperse giant unilamellar vesicles have a diameter measured by confocal microscopy between 5 µm and 30 µm.

In a preferred embodiment, the method for preparing monodisperse giant unilamellar vesicles using the device (1) for water-in-air generation of droplets comprises the following steps:
a) providing a core-annular liquid-liquid flow comprising an aqueous core solution and a water-immiscible organic shell solution, wherein the water-immiscible organic shell solution comprises a volatile organic solvent and a first lipid,
b) acoustically modulating the core-annular liquid-liquid flow, thereby forming a flow of core-shell droplets comprising a core consisting of the aqueous core solution and a shell consisting of the water-immiscible organic shell solution, wherein the first lipid forms a monolayer around the droplet core,
c) at least partially evaporating the volatile organic solvent from the flow of core-shell droplets,
d) submerging the flow of core-shell droplets in a rotating dispersion to produce monodisperse giant unilamellar vesicles,

wherein in step d) the rotating dispersion comprises an aqueous phase and a second lipid, wherein the second lipid is arranged as a monolayer on the surface of the aqueous phase so that the monolayer of the second lipid encloses the core-shell droplets thereby forming a double layer with the monolayer of the first lipid, and
wherein in step d) the rotating dispersion exhibits a rotational laminar flow profile which is synchronized with the flow rate of the core-shell droplets, and
wherein the monodisperse giant unilamellar vesicles are prepared in a frequency between 5,000 vesicles per second and 30,000 vesicles per second.

In a preferred embodiment, the method for preparing monodisperse giant unilamellar vesicles using the device (1) for water-in-air generation of droplets comprises the following steps:
a) providing a core-annular liquid-liquid flow comprising an aqueous core solution and a water-immiscible organic shell solution, wherein the water-immiscible organic shell solution comprises a volatile organic solvent and a first lipid,
b) acoustically modulating the core-annular liquid-liquid flow, thereby forming a flow of core-shell droplets comprising a core consisting of the aqueous core solution and a shell consisting of the water-immiscible organic shell solution, wherein the first lipid forms a monolayer around the droplet core,
c) at least partially evaporating the volatile organic solvent from the flow of core-shell droplets,
d) submerging the flow of core-shell droplets in a rotating dispersion to produce monodisperse giant unilamellar vesicles,

wherein in step d) the rotating dispersion comprises an aqueous phase and a second lipid, wherein the second lipid is arranged as a monolayer on the surface of the aqueous phase so that the monolayer of the second lipid encloses the core-shell droplets thereby forming a double layer with the monolayer of the first lipid, and
wherein in step d) the rotating dispersion exhibits a rotational laminar flow profile which is synchronized with the flow rate of the core-shell droplets, and
wherein the first lipid and the second lipid are independently of each other selected from the group consisting of:
   a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
   an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidyl inositol phosphates, cardiolipins, bis(monoacylglycero)phosphate derivatives;
   a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-dioleoyl-3-dimethylammonium-propane;
   a pH-sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-snglycero-3-succinate, N-palmitoyl homocysteine;
   a photoswitchable lipid;
   acylglycine derivatives, prenol derivatives, prostaglandine derivatives, glyco-sylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
   one of the aforementioned lipids coupled to a functional ligand selected from biotin, N-hydroxysuccinimide (NHS) ester, sulfo- NHS ester, nitrilotriacetic acid -nickel, amine, carboxylic acid, maleimides, dithiopyridinyl, pyridyl disulfide, pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator; and
   one of the aforementioned lipids coupled to polyethyleneglycol with a molecular weight comprised between 350 and 50,000 g/mol.

Another aspect is directed to a method for preparing a three-dimensional microenvironment niche for a cell or a synthetic cell using the device (1) for water-in-air generation of droplets according to the invention. Thus, the present invention is also directed to a method for preparing a three-dimensional microenvironment niche for a cell or a synthetic cell using the device (1) for water-in-air generation of droplets comprising the following steps:
a) providing a liquid flow comprising a cell and/or a synthetic cell, and a first cell culture medium comprising a photocrosslinkable polymer hydrogel, and a photocatalyst,
b) acoustically modulating the liquid flow, thereby forming a flow of droplets comprising the cell and/or the synthetic cell encapsulated in the first cell culture medium,
c) irradiating the droplets to photochemically induce a crosslinking of the photocrosslinkable polymer hydrogel thereby gelling the droplet
d) collecting the flow of gelled droplets in a second cell culture medium to produce a three-dimensional microenvironment niche.

In a preferred embodiment, the method for preparing a three-dimensional microenvironment niche for a cell or a synthetic cell using the device (1) for water-in-air generation of droplets comprises the following steps:
a) providing a liquid flow comprising a cell and/or a synthetic cell, and a first cell culture medium comprising a photocrosslinkable polymer hydrogel, and a photocatalyst,
b) acoustically modulating the liquid flow, thereby forming a flow of droplets comprising the cell and/or the synthetic cell encapsulated in the first cell culture medium,
c) irradiating the droplets to photochemically induce a crosslinking of the photocrosslinkable polymer hydrogel thereby gelling the droplet
d) collecting the flow of gelled droplets in a second cell culture medium to produce a three-dimensional microenvironment niche,
wherein in step d) the second cell culture medium is agitated.

In a preferred embodiment, the method for preparing a three-dimensional microenvironment niche for a cell or a synthetic cell using the device (1) for water-in-air generation of droplets comprises the following steps:
a) providing a liquid flow comprising a cell and/or a synthetic cell, and a first cell culture medium comprising a photocrosslinkable polymer hydrogel, and a photocatalyst,
b) acoustically modulating the liquid flow, thereby forming a flow of droplets comprising the cell and/or the synthetic cell encapsulated in the first cell culture medium,
c) irradiating the droplets to photochemically induce a crosslinking of the photocrosslinkable polymer hydrogel thereby gelling the droplet
d) collecting the flow of gelled droplets in a second cell culture medium to produce a three-dimensional microenvironment niche,
wherein in wherein the photocrosslinkable polymer hydrogel is a polymer chemically functionalized with one or more methacrylate groups selected from the group consisting of: hyaluronic acid, chitosan, dextran, heparin, alginate, gelatin, fibrin, collagen type I, collagen type II, collagen type III, collagen type IV, fibronectin, vitronectin, tropoelastin, laminin, silk, sericin, polyvinyl alcohol, polyethylene glycol, sodium polyacrylate, acrylate polymers and copolymers thereof.

In a preferred embodiment, the method for preparing a three-dimensional microenvironment niche for a cell or a synthetic cell using the device (1) for water-in-air generation of droplets comprises the following steps:
a) providing a liquid flow comprising a cell and/or a synthetic cell, and a first cell culture medium comprising a photocrosslinkable polymer hydrogel, and a photocatalyst,
b) acoustically modulating the liquid flow, thereby forming a flow of droplets comprising the cell and/or the synthetic cell encapsulated in the first cell culture medium,
c) irradiating the droplets to photochemically induce a crosslinking of the photocrosslinkable polymer hydrogel thereby gelling the droplet
d) collecting the flow of gelled droplets in a second cell culture medium to produce a three-dimensional microenvironment niche,
wherein the photocatalyst is a Ru(II) complex.

### Description of the Figures

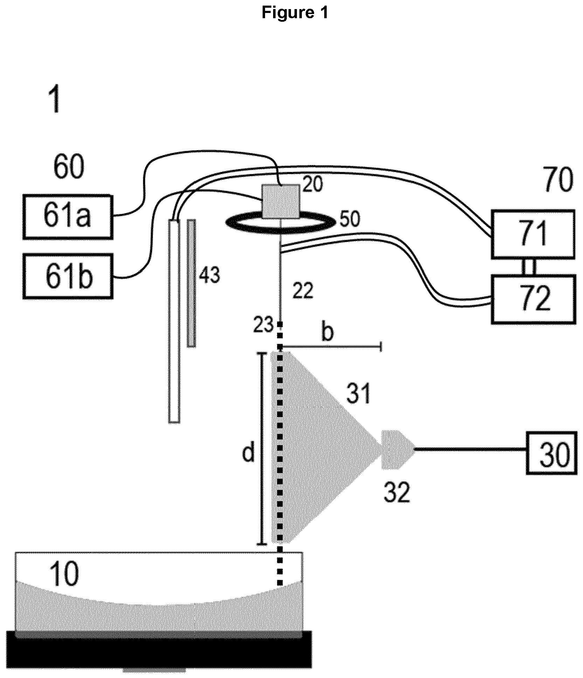

- **Figure 1**: shows an exemplary embodiment of the inventive device (1) for water-in-air generation of droplets comprising: a collection vessel (10), a droplet generation device (20) comprising a piezoelectric transducer (21), a self-centering mount (50), and a capillary (22) with a nozzle (23) pointing downwards to the collection vessel; a laser source (30) generating a visible light laser beam (31) with tophat profile by a beam shaping optic (32), an imaging setup for continuous monitoring the droplet generation (40) comprising a high-speed camera (41), a microscope lens (42) and a backlight (43); and a liquid delivery system (60) comprising two syringe pumps (61a, 61b).
- **Figure 2**: shows an exemplary embodiment of the rotation mount (81) of the inventive device (1) comprising ball bearing (81), a belt (82) connecting the ball bearing (81) with a drive (83). Rotational speed may be adjusted with a dial at the drive (83).
- **Figure 3**: shows in a side view an exemplary embodiment of the droplet generation device (20) of the inventive device (1) comprising a piezoelectric transducer (21), a capillary (22) with a nozzle at the bottom end (23), a mounting block (24), and the self-centering mount (50).
- **Figure 4**: shows in a top view an exemplary embodiment of the self-centering mount (50) of the inventive device (1).
- **Figure 5**: shows a side view of an examplary capillary configuration of the droplet generation device (20) of the inventive device (1) comprising two capillaries (22a, 22b) nested into each other for producing core-shell droplets.

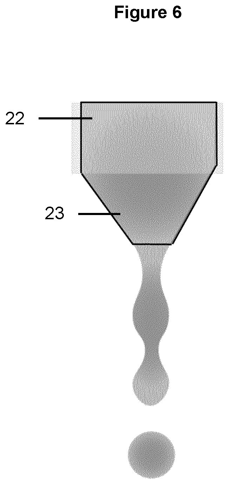

- **Figure 6**: shows a side view of an examplary capillary configuration of the droplet generation device (20) of the inventive device (1) comprising a single capillary (22) with a nozzle (23) at the bottom.

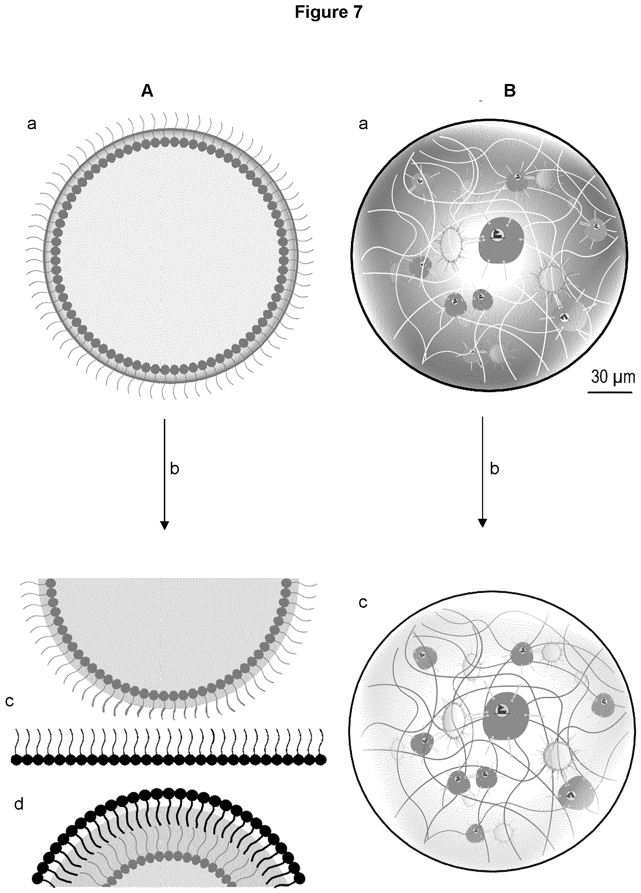

- **Figure 7**: shows two applications of the inventive device (1): (A) Preparation of monodisperse giant unilamellar vesicles with the inventive device (1): a) core-shell droplet formation by droplet generation device (20) with an aqueous core and shell consisting of a solvent and a lipid arranged as monolayer, b) evaporation of solvent shell as droplets transit in air towards the collection vessel (10) c) collecting the droplets in a rotating dispersion of a lipid in an aqueous solution, d) the droplet submerges into the dispersion, thereby a monodisperse GUV is formed; (**B**) Preparation of three-dimensional microenvironment niche for a cell or a synthetic cell a) droplet with an encapsulated cell and/or synthetic cell is formed by the droplet generation device (20); b) droplet is irradiated by a laser to induce gellation of the droplet, c) gelled droplet is collected in a bioreactor with a cell culture medium.
- **Figure 8**: shows a diagram characterizing core-annular liquid-liquid flow profiles as the ratio of the flow rate of the inner liquid to the outer liquid and the impact on the produced droplet diameter at two acoustic driving amplitudes of 100 V (circles) or 300 V (squares) at different time points of during in-air production (black), immediately after collection (grey), and 24 hours after collection (ligh grey). For proof of principle, these characterizations are completed using water as the core phase and fluorinated oil with commercially available fluorosurfactant in the annular. Error bars represent the standard deviation from the mean with the source of error coming from the occasional generation of satellite droplets, or smaller droplets formed at the droplet breakoff location in the flow stream due to unsynchronized flow profile with the driving frequency of the acoustic signal.
- **Figure 9**: **(A)** shows a photomicrograph from high-speed camera of the nozzles with core-annular liquid-liquid flow profile exiting the nozzle at the flow rate ratio of 1 and typical "figure 8" modulation impact by the acoustic field imparting a 10 kHz 300 V sine wave. The droplet breakoff point is clearly displayed with single-file droplets in transit in the air. Scale bar is 150 µm; (**B**) shows a photomicrograph of droplets collected in fluorinated oil immediately after in-air generation. Scale bar is 100 µm; (C) shows a photomicrograph of droplets collected in fluorinated oil after 24 hours incubation at room temperature, demonstrating a lack of droplet coalescence and that in-air droplet generation is stable with current commercially available fluorosurfactants. Scale bar is 100 µm.

The following examples are included to demonstrate preferred embodiments of the invention. It should be appreciated by those of skill in the art that the techniques disclosed in the following examples represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the scope of the invention as described in the following claims.

### Examples

### Example 1: Exemplary materials and methods for synthetic cell and niche formation:

The device and apparatus for generating giant unilamellar vesicles for the purpose of synthetic cell formation is built and mounted as described in the application, using an inner nozzle with 5 µm diameter and outer nozzle with 10 µm diameter. This two-channel device design uses fluid injection from two separate reservoirs whereby the core aqueous fluid comprises a biological buffer and the sheath fluid comprises chloroform with a suspension of lipids. The fluid phases are prepared using chemicals purchased from Sigma-Aldrich Chemie GmbH (Taufkirchen, Germany) unless otherwise noted. For the purpose of this description, the simplest implementation of synthetic cell formation will be described. It is important to note that from this foundational protocol, higher complexity may be engineered into the system. The aqueous buffer is either commercially available PBS, 25 mM HEPES, 10 mM TRIS, or 20 mM PIPEs depending on the potential to include a range of biomolecules with different buffering capacity needs in the core phase of the droplets. The sheath phase is prepared by suspending 6 mM total concentration of desired lipid composition in chloroform at -20 °C. For the purpose of this discussion, we take 25 mg/mL DOPC in chloroform from the distributor and adjust the concentration to achieve 6000 µM. These suspensions are loaded in the fluidic reservoirs, keeping the aqueous solution at room temperature and the sheath solution on ice. In the meantime, the collection bath is prepared by adding a suspension of 10 mg/mL DOPC in chloroform dropwise to the surface of 100 mL of MilliQ water in the Teflon collection vessel until a uniform spreading has occurred. The choloroform is allowed to evaporate, leaving a monolayer of lipids at the air-water interface and the Teflon vessel is secured on to the rigid body rotation platform.

To generate core-sheath droplets, the dead volumes of the device channels are eliminated and the appropriate flow rate ratio for the generation of 20 µm diameter droplets in air is dialed in to the pump and piezoelectronic controls. The rigid body rotation platform is then positioned below the device mount such that there is an air gap of 40 cm and the droplet landing postion is set to within 1 cm from the edge of the collection vessel where it is possible to achieve a circumferential velocity of roughly 1 m/s with a rotational laminar flow profile. The calibration of this device is dependent on the diameters of the gears and the length of the belt controlling the bearings. It should be synchronized to the fluid velocity of the generated droplets, taking into account the effect of piezoelectric actuation. For syringe pumps injecting the core/sheath fluids, we use approximately 0.8 mL/min core flow rate and 1.0 mL/min sheath flow rate while the piezoelectronic tube is actuated at 10 kHz and 10 Vp-p amplitude. The droplets are generated in-air with an initial air-chloroform/lipids sheath-water core layering. As the droplets transit in air, the volatile chloroform begins to evaporate and the lipids form a layer at the emerging air-water interface with the polar head groups facing towards the water and the hydrophobic tails facing outwards. In the collection vessel, the surface of the rotating fluid likewise has lipid orientation such that as the droplets begin to arrive at the surface of the rotating collection vessel, the lipids form a bilayer during the droplet submersion, completing the formation of GUVs by the described mechanism.

Examples of engineering further complexity to this method includes encapsulating recombinant protein systems to the core of the droplets by suspending the proteins into relevant biological buffers. The efficiency of GUV formation can also be tracked by using fluorescent lipids in the lipid composition. The size of these lipid-based compartments is also highly tunable depending on the fluid dynamics parameters used in the operation of the apparatus.

The device and apparatus for generating synthetic niches for the purpose of encapsulating stem cells in controllable 3D droplet-templated microenvironments is built and mounted as described in the application, using a single nozzle with a diameter of at least 40 µm depending on the desired final droplet diameter. This single-channel device design uses fluid injection from a single reservoir whereby the aqueous fluid comprises a pre-gel fluid with suspended cells/synthetic cells in appropriate cell culture media with methacrylate-functionalized polymers and ruthenium compound. The methacrylate-functionalized polymers, using PEGDA for discussion purposes, and ruthenium compound in cell culture media is prepared according to the manufacturer's specifications (Advanced BioMatrix, Carlsbad, CA, USA). All cell culture consumable supplies are acquired froom ThermoFisher Scientific, Norristown, PA, USA unless otherwise noted. Briefly, working in the dark, 1 mL of pre-gel solution is prepared in StemFlex cell culture media by allowing the PEGDA to equilibrate to room temperature. Then, 100 mg is added to the media with gentle pipetting to mix and filtered using a 0.2 µm syringe filter. The hiPSCs are cultured using standard hiPSC stem cell culture protocols and conditions and processed into a pellet at the desired cell density (approximately 1e5 cells/mL). The cells are resuspended in the pre-gel suspension followed by addition of 20 µL each of solubilized ruthenium and sodium persulfate in PBS so that the final concentrations are 37.4 mg/mL and 119 mg/mL, respectively, and gently pipetted to finalize the solution preparation. The collection bath is prepared using StemScale PSC Suspention Medium at 37 °C and aligned below the device on top of a magnetic stir plate with gentle agitation.

The pre-gel solution is loaded into a pressure-rated Hamiltion syringe (Hamilton Company, Reno, NV, USA) and injected through the device at a flow rate of 0.5 mL/min (a parameter that is tuned depending on the viscosity of the pre-gel solution). Simultaneously, the apparatus is initiated such that the piezoelectronic tube is actuated using a sine wave at 15 kHz with 10 Vp-p while the 10 cm-long laser line is likewise directly applied to the droplet stream with up to 15 W power, aligned below the droplet breakoff point of the stream fluid profile. Upon irradiation, the photochemical reaction occurs and crosslinks the methacrylated groups on the backbone of the PEDGA polymers forming the droplets. As the droplets transit through the laser line, the power applied and droplet time of flight can be tuned to control the degree of crosslinking, ie desired stiffness, of the produced gelled droplets. The collected cell-encapsulating gel spheres are then transferred to spinner-flask bioreactor culture using standard conditions and evaluated for a range of post-production biological parameters such as fluoresence assays for determining stem cell plasticity, viability, and differentiation.

### Example 2: Exemplary construction of a device (1) for water-in-air generation of droplets

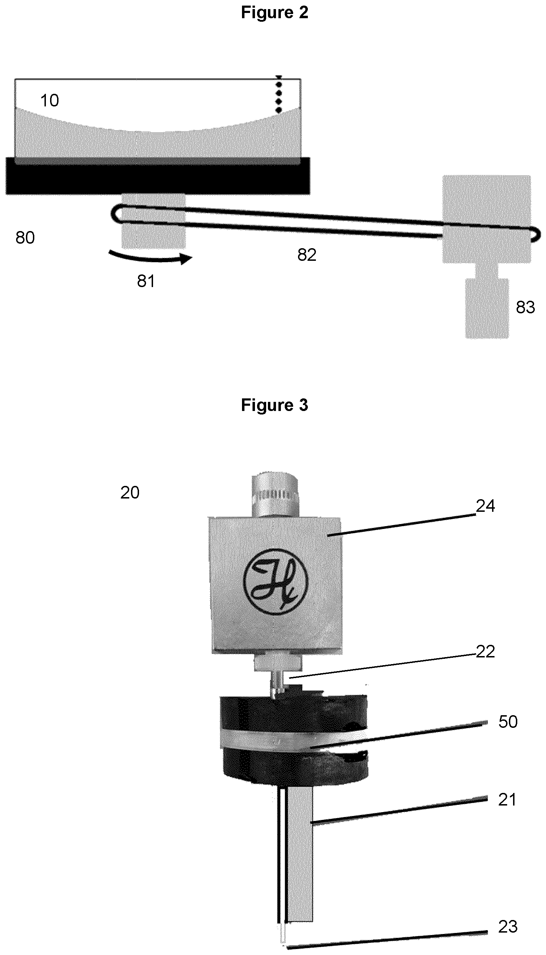

The device (1) for water-in-air generation of droplets is constructed using optomechanical components purchased from ThorLabs Inc. (Newton, NJ, USA) unless otherwise noted. An UltraLight Breadboard, 300 mm x 450 mm x 25 mm, M6 Tap is fixated onto an optical table (Integrity 2 VCS, Newport Corporation, Darmstadt, Germany) with added sorobotane feet for vibration dampening. The apparatus is built using posts and post holders to mount the devices and produced fluidic droplets within an image relay space that is designed for vertical operation of the devices with enough space such that the collection modules (RBR or agitation) can be installed within the space below the image relay and optical path for the tophat laser line. The laser line is aligned so that the beam profile is free of obstructions and aimed at a beam dump to collect stray light emitted from the high-power laser module (FS30-B with power supply, OptLasers, Bia 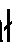 ystok, Poland) that is connected to the beam shaping optic using a fiber optic cable (Custom Compact Beam Shaper Kit, Limo GmbH, Dortmund, Germany). Each module is further aligned using micromanipulators for the device mount, backlighting LED/diffuser, high-speed camera (MicroC211, Phantom High-speed, Wayne, NJ, UAS) and objective (5x, Zeiss, Oberkochen, Germany), and the beam shaping optic. The fluidic injection system is also organized on the breadboard and is operated depending on the application with either syringe pumps, peristaltic pumps, or pressure driven flow apparatuses to include appropriate tubing and fittings (Darwin Microfluidics, Paris, France). The piezoelectric block is connected to a waveform generator (Tektronix AFG31022, Germany) and amplifier (AG 1021, TC Power Conversion, Rochester, NY, USA) by standard BNC cables and connectors. The entire apparatus is housed within a custom-designed optical breadboard enclosure that is functionalized with safety switches (Sensors RSS260 and Safety Relay SRB-E-301 ST, Schmersal GmbH, Wuppertal, Germay) for controlling the operation of the high-power laser and connected to a Dell PC with appropriate software to control the high-speed camera. Finally, the collection vessel is installed on a rigid body rotating (RBR) mount or equipped with a stirrer (e.g. a magnetic stir plate in the appropriate size for the collection volume (for example, IKA Magnetic Stirrer-RH Digital, IKA-Werke GmbH, Staufen, Germany)). The RBR is constructed OEM with a custom-built Teflon trough with a desired diameter and wall height appropriate for the droplet application that is secured to the RBR platform. The apparatus details are in Figs. 1 and 2.

### Example 3: Exemplary construction of a droplet generation device (20) of the inventive device (1) for water-in-air generation of droplets

The self-centering device mount (50) is fabricated OEM using a stainless-steel cube with ports bored out in a 4-way intersection through the cross-section of the block. Finger-tight ¼-28" threaded fittings (Hamilton Company, Reno, NV, USA), compatible with glass capillaries that are arranged and mounted according to the number of solutions to be delivered into the device. The capillaries, ferules, and fittings are assembled within the intersecting ports of the block to achieve appropriate phase chemistry orientations that precisely controls the dispensing of the fluids for the disclosed applications. The fittings are compatible with glass capillaries of dimensions: (i) 1 mm O.D. x 0.58 mm I.D.; (ii) 2 mm O.D. x 1.56 mm I.D. (World Precision Instruments, Sarasota, FL, USA) that are manually fabricated into nozzles using a capillary pipette puller (PUL-1000, World Precision Instruments, Sarasota, FL, USA) and microforge instrument (MF-2, Narishige, Japan) to form size-controlled nozzle diameters ranging from 5 to 100 µm. Appropriate tubing such as Tygon, Silastic, or PEEK is attached to the inner and/or outer fluidic channels to inject the solutions into the device during operation. The injection is driven by the use of either syringe pumps, peristaltic pumps, or pressure driven flow apparatuses depending on the fluid dynamics of the respective droplets being produced (see figure 3).

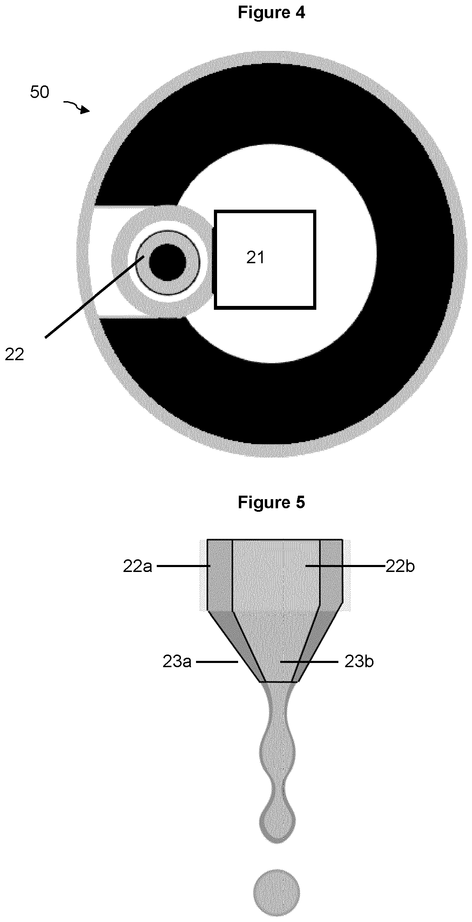

The mounting block construction (24) is then nested within a custom-built piezoelectric transducer block that is designed for repeated use of piezoelectric crystalline materials (Physik Instrumente GmbH & Co. KG, Karlsruhe, Germany) with specifications sourced for operation in the kHz frequency domain, mounted onto a stainless-steel capillary (Custom 2.1 mm I.D. tubing, 3 cm length, World Precision Instruments, Sarasota, FL, USA) to nest the mounting block, that is then installed onto the final apparatus using a self-modified rubber stopper (see figure 4).

### Example 4: Preparation of giant unilamellar vesicles as synthetic cells

Chemicals were purchased from Sigma-Aldrich Chemie GmbH (Taufkirchen, Germany). The outer phase that is loaded into the injection port of the device (1) for the templated formation of synthetic cells comprises lipids of a desired composition suspended in chloroform at least -20 °C in temperature when loaded into the fluidic injection channel. The inner water phase for the formation of synthetic cells is prepared by creating a biological aqueous buffer appropriate for the category of synthetic cell to be generated. The category of synthetic cell to be generated includes, but is not limited to, GUVs with ranges of lipid compositions, GUVs with encapsulated protein cargoes, GUVs with membrane pores or proteins or transmembrane proteins, GUVs with encapsulated transcription-translation (TX-TL) systems, GUVs with DNA or RNA origami systems, GUVs with encapsulated natural or synthetic organelles, and GUVs with synthetic signaling pathways components. For each category, the inner water phase biological buffer is prepared accordingly. The phase chemistry in the collection vessel for the formation of synthetic cells is prepared using only the aqueous buffering salt conditions and may contain a density and/or viscosity tuning agent to balance the fluid dynamics and osmolarity of the collection solution against the GUVs.

### Example 5: Preparation of three-dimensional microenvironment niches

All chemicals purchased from Advanced BioMatrix, Carlsbad, CA, USA. For the formation of synthetic niche microenvironments, the phase chemistry can be prepared in a variety of water-based or oil-free approaches that ultimately produces a hydrogel sphere in an aqueous suspension. For a single-channel device designs, the pre-gel fluid is prepared by dissolving water-soluble polymers chemically functionalized with methacrylate groups and ruthenium compound for the basis of the photochemistry reaction that will polymerize during droplet generation. If cargo is intended to be encapsulated within the hydrogel-based synthetic niche, then cells and/or synthetic cells are added to the solution, including to contain appropriate cell culture media formulations.

In dual-channel device designs, aqueous two-phase systems (ATPS) are implemented. For example, high concentrations of two different high molecular weight polymers, such as polyethylene glycol or polyvinyl alcohol or dextran etc., are mixed within a separatory funnel to form an initial emulsion that is allowed to separate into an enriched equilibrium point that is determined by binodal characterization and analysis. That is, each polymer phase separates into an enriched top and bottom phase with enriched-phase and dilute-phase polymer concentrations dependent on the final volume of the collected phase in question. When injected into the droplet generating apparatus in a two-channel device design, these aqueous-two phase systems generate water-in-water droplets. The core water droplet comprises the photochemistry hydrogelation system along with cargo to be encapsulated whereas the surrounding shell water phase works to stabilize the pre-gel droplet for the photochemistry kinetics to produce monodisperse and spherical hydrogels with sizes up to 100 µm diameter. These synthetic niche microenvironments templated by the hydrogel sphere are collected and cultured in bioreactors.

### Reference Signs

- 1: device for water-in-air generation of droplets
- 10: collection vessel

- 20: droplet generation device
- 21: piezoelectric transducer
- 22: at least one capillary
- 23: nozzle
- 24: mounting block

- 30: laser source
- 31: visible light laser beam
- 32: beam shaping optic

- 40: imaging setup for continuous monitoring the droplet generation
- 41: high-speed camera
- 42: microscope lens
- 43: backlight

- 50: self-centering mount

- 60: liquid delivery system
- 61: pump system
- 61a,61b: syringe pump
- 62: at least one liquid reservoir

- 70: feedback controller

- 80: rotation mount
- 81: ball bearing
- 82: belt
- 83: drive

## Claims

1. A device (1) for water-in-air generation of droplets comprising:
- a collection vessel (10) comprising an opening on top
- a droplet generation device (20) positioned above the collection vessel (10) comprising a piezoelectric transducer (21) and at least one capillary (22) with a nozzle (23) pointing downwards to the collection vessel; and
- a laser source (30) generating a visible light laser beam (31) with tophat profile,
- an imaging setup for continuous monitoring the droplet generation (40) comprising a high-speed camera (41), a microscope lens (42) and a backlight (43);
wherein the piezoelectric transducer (21) is configured to acoustically generate a flow of droplets passing from the nozzle (23) to the collection vessel (10), and wherein the laser beam (31) is aligned and focused on said flow of droplets passing from the nozzle (23) to the collection vessel (10) to induce a photochemical reaction in the droplets.

2. The device (1) according to claim 1, wherein the droplet generation device (20) is mounted on a self-centering mount (50).

3. The device (1) according to claim 1 or 2, wherein the device (1) further comprises a liquid delivery system (60) comprising a pump system (61) and at least one liquid reservoir (62), wherein the liquid delivery system (60) delivers liquid(s) from the at least one liquid reservoir (62) to the at least one capillary (22).

4. The device (1) according to any one of claims 1 to 3, wherein the piezoelectric transducer (21) is designed as a vertically arranged tube surrounding the at least one capillary (22) with the nozzle (23), thereby the nozzle (23) is located in an acoustic field generated by the piezoelectric transducer (21).

5. The device (1) according to any one of claims 1 to 4, wherein the device (1) further comprises a feedback controller (70) operatively coupled to the imaging setup for continuous monitoring the droplet generation (40) and the piezoelectric transducer (21), wherein the feedback controller (70) is configured to control the piezoelectric transducer (21).

6. The device (1) according to any one of claims 1 to 5, wherein the vertical distance d between the nozzle (23) and the collection vessel (10) is with at least 40 mm large enough to allow volatile components to evaporate from the flow of droplets.

7. The device (1) according to any one of claims 1 to 5, wherein the at least one capillary (22) comprises a first capillary (22a) with a first cavity (24a) and a second capillary (22b) with a second cavity (24b), wherein the second capillary (22b) has a smaller diameter than the the first capillary (22a) and the second capillary (22b) is nested in the first capillary (22a).

8. The device (1) according to any one of claims 1 to 7, wherein the collection vessel (10) is mounted on a rotation mount (80), wherein the rotational speed of the rotation mount (80) is adjustable to generate a rotational laminar flow profile of a collection liquid in the collection vessel (10) towards the edge of the collection vessel which is synchronized with the droplet flow rate.

9. A method for preparing monodisperse giant unilamellar vesicles using the device (1) according to any one of claims 1 to 8 comprising the following steps:
a) providing a core-annular liquid-liquid flow comprising an aqueous core solution and a water-immiscible organic shell solution, wherein the water-immiscible organic shell solution comprises a volatile organic solvent and a first lipid,
b) acoustically modulating the core-annular liquid-liquid flow, thereby forming a flow of core-shell droplets comprising a core consisting of the aqueous core solution and a shell consisting of the water-immiscible organic shell solution, wherein the first lipid forms a monolayer around the droplet core,
c) at least partially evaporating the volatile organic solvent from the flow of core-shell droplets,
d) submerging the flow of core-shell droplets in a rotating dispersion to produce monodisperse giant unilamellar vesicles,
wherein in step d) the rotating dispersion comprises an aqueous phase and a second lipid, wherein the second lipid is arranged as a monolayer on the surface of the aqueous phase so that the monolayer of the second lipid encloses the core-shell droplets thereby forming a double layer with the monolayer of the first lipid, and
wherein in step d) the rotating dispersion exhibits a rotational laminar flow profile which is synchronized with the flow rate of the core-shell droplets.

10. The method according to claim 9, wherein the monodisperse giant unilamellar vesicles have a diameter measured by confocal microscopy between 5 µm and 30 µm.

11. The method according to claim 9 or 10, wherein the monodisperse giant unilamellar vesicles are prepared in a frequency between 5,000 vesicles per second and 30,000 vesicles per second.

12. The method according to any one of claims 9 to 11, wherein the first lipid and the second lipid are independently of each other selected from the group consisting of:
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether;
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidyl inositol phosphates, cardiolipins, bis(monoacylglycero)phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3-dioleyloxy)propyl)-N,N,N-trimethylammonium chloride; 3β-(N-(N',N'-dimethyl-aminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-dioleoyl-3-dimethylammonium-propane;
a pH-sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine;
a photoswitchable lipid;
acylglycine derivatives, prenol derivatives, prostaglandine derivatives, glyco-sylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from biotin, N-hydroxysuccinimide ester, sulfo-hydroxysuccinimide ester, nitrilotriacetic acid -nickel, amine, carboxylic acid, maleimides, dithiopyridinyl, pyridyl disulfide, pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator; and
one of the aforementioned lipids coupled to polyethyleneglycol with a molecular weight comprised between 350 and 50,000 g/mol.

13. A method for preparing a three-dimensional microenvironment niche for a cell or a synthetic cell using the device (1) according to any one of claims 1 to 8 comprising the following steps:
a) providing a liquid flow comprising a cell and/or a synthetic cell, and a first cell culture medium comprising a photocrosslinkable polymer hydrogel, and a photocatalyst,
b) acoustically modulating the liquid flow, thereby forming a flow of droplets comprising the cell and/or the synthetic cell encapsulated in the first cell culture medium,
c) irradiating the droplets to photochemically induce a crosslinking of the photocrosslinkable polymer hydrogel thereby gelling the droplet
d) collecting the flow of gelled droplets in a second cell culture medium to produce a three-dimensional microenvironment niche.

14. The method according to claim 13, wherein in step d) the second cell culture medium is agitated.

15. The method according to claim 13 or 14, wherein the photocrosslinkable polymer hydrogel is a polymer chemically functionalized with one or more methacrylate groups selected from the group consisting of: hyaluronic acid, chitosan, dextran, heparin, alginate, gelatin, fibrin, collagen type I, collagen type II, collagen type III, collagen type IV, fibronectin, vitronectin, tropoelastin, laminin, silk, sericin, polyvinyl alcohol, polyethylene glycol, sodium polyacrylate, acrylate polymers and copolymers thereof.
